Europäisches Patentamt

European Patent Office

Office européen des brevets

(11) Publication number: **0 124 613 B1**

## EUROPEAN PATENT SPECIFICATION

(12)

(45) Date of publication of patent specification: **30.12.92**

(21) Application number: **83903315.6**

(22) Date of filing: **27.10.83**

(86) International application number:
**PCT/JP83/00386**

(87) International publication number:
**WO 84/01714 (10.05.84 84/12)**

(51) Int. Cl.⁵: **A61K 37/54**, A61K 35/44,
A61K 35/23

The file contains technical information submitted
after the application was filed and not included in
this specification

㊹ NOVEL PLASMINOGEN ACTIVATOR DERIVED FROM HUMAN KIDNEYS, PROCESS FOR ITS
PREPARATION, AND THROMBOLYTIC DRUG CONTAINING THE SAME.

(30) Priority: **29.10.82 JP 189065/82**
**29.10.82 JP 189067/82**

(43) Date of publication of application:
**14.11.84 Bulletin 84/46**

(45) Publication of the grant of the patent:
**30.12.92 Bulletin 92/53**

(84) Designated Contracting States:
**BE FR**

(56) References cited:
**EP-A- 41 766**
**EP-A- 99 126**

**CHEMICAL ABSTRACTS, vol. 92, no. 17, 28
April 1980, Columbus, OH (US); E.L.WILSON
et al., p. 413, no. 144679a**

(73) Proprietor: **MITSUI TOATSU CHEMICALS, Inc.**
**2-5 Kasumigaseki 3-chome**
**Chiyoda-Ku Tokyo 100(JP)**

(72) Inventor: **TAKAHASHI, Seishi**
**1071-2, Nakano-cho Totsuka-ku**
**Yokohama-shi Kanagawa 247(JP)**
Inventor: **MORIMOTO, Kazushi**
**4-1409-933, Shiomidai 1-chome Isogo-ku**
**Yokohama-shi Kanagawa 235(JP)**
Inventor: **FUJISAWA, Yoshikazu**
**11-2, Tamanawa 5-chome Kamakura-shi**
**Kanagawa 247(JP)**
Inventor: **IKEDA, Fumiaki**
**2882, Iijima-cho Totsuka-ku**
**Yokohama-shi Kanagawa 244(JP)**

(74) Representative: **Behaghel, Pierre et al**
**CABINET PLASSERAUD 84 rue d'Amsterdam**
**F-75009 Paris(FR)**

CHEMICAL ABSTRACTS, vol. 76, no. 5, 31 January 1972, Columbus, OH (US); A.NOBUO et al., p. 134, no. 22330d

KETSUEKI TO MYAKUKAN, vol. 13, no. 3, September 1982; K.SUEISHI et al., pp. 347-350

BIOCHIMICA ET BIOPHYSICA ACTA, vol. 717, no. 2, August 1982; K.SUEISHI et al., pp. 327-336

BIOCHIMICA ET BIOPHYSICA ACTA, vol. 621, 1980; B.AASTED, pp. 241-254

BIOCHIMICA ET BIOPHYSICA ACTA, vol. 580, 1979; D.C.RIJKEN et al., pp. 140-153

JOURNAL OF BIOLOGICAL CHEMISTRY, vol. 254, no. 6, 25 March 1979; B.R.BINDER et al., pp. 1998-2003

JOURNAL OF BIOLOGICAL CHEMISTRY, vol. 256, no. 13, 10 July 1981; D.C.RIJKEN et al., pp. 7035-7041

JOURNAL OF BIOLOGICAL CHEMISTRY, vol. 255, no. 8, 25April 1980; D.VETTERLEIN et al., pp. 3665-3672

## Description

Technical Field:

This invention relates to a novel plasminogen activator derived from human kidney, its preparation process and a thrombolytic agent containing the same as an active ingredient.

Background Art:

Plasminogen activators may be classified, in accordance with their sources, into tissue activators, vascular activators, blood activators, urokinase, and the like. Plasminogen activators play an important role in fibrinolytic (fibrin-decomposing) activities and are found in a variety of organs of mammalian animals. On the other hand, a great deal of work has recently been made to obtain plasminogen activators from cultured cells. In the above work, there were used as cultivated cells, for example, malignant tumor cells, blood vessel endothelium, stimulated macrophage, granular starch cells stimulated by follicle-stimulating hormone, and the like. There have also been reported biochemical and immunological properties of plasminogen activators isolated from humoral tissue homogenates, cultivated cells and culture media.

However, the purification of human tissue plasminogen activator has seldom been carried out. This seems to be attributed to the difficulty in obtaining the starting material in a sufficient amount and, besides, the hydrophobic property of enzymes contained in the starting material and the instability of such enzymes in a buffer and, in some instances, the existence of unknown protease in the course of its purification. Under these circumstances, Rijken et al isolated human uterus plasminogen activator having a molecular weight of 69,000 and confirmed that the activator is similar both immunologically and biochemically to human vascular plasminogen activator. In addition, another human vascular plasminogen activator having a molecular weight different from the above human uterus plasminogen activator has also been isolated and identified.

Kwaan et al carried out an investigation on a plasminogen activator derived from human kidney tissue and revealed that the activator is present principally in the endothelium of blood vessels, especially in the endothelium of veins [Fed. Proc. 24, 387 (1965)]. It has also been reported by Kucinski et al [J. Clin. Invest. 47, 1238-1253 (1968)], Bernik et al [J. Clin. Invest. 48, 1740-1753 (1969)], Barlow et al [Thromb. Res. 1, 201-208 (1972)], Åsted et al [Experimentia 33, 589-590 (1978)] and Lewis [Thromb. Haemostas. 42, 895-890 (1979)] that such a plasminogen activator obtained from the culture of human kidney tissue is identical immunologically and physiochemically to urokinase.

Urokinase, which is a plasminogen activator isolated from urine or cultivated kidney cells, and streptokinase, which is a plasminogen activator collected from streptococci, are presently used as thrombolytic agents for the purpose of treating thrombosis. However, both urokinase and streptokinase are different from the normal plasminogen activator collected from blood. These plasminogen activators do not have any specific affinity to fibrin. Thus, the results obtained from the use of urokinase and streptokinase in the treatment of thrombosis were not fully satisfactory in every aspect. In order to obtain desired effects, it is necessary to administer them in relatively large amounts. However, massive administration induces such dangerous side effects as resolution of fibrinogen and internal bleeding. There is thus a strong outstanding demand for the development of a drug which may be prepared on a relatively large scale, have little side effects and enjoy high thrombus-resolving activity.

As a result of an intensive investigation on the tissue plasminogen activator isolated and purified from human kidneys and human blood vessels, the present inventors have surprisingly found a plasminogen activator having properties significantly different from those of the urokinase derived from human kidneys or human blood vessels, although it has been believed that urokinase is only the principal plasminogen activator available from human kidneys and human blood vessels.

In the course of a further investigation on the plasminogen activator different from urokinase and derived from human kidney, relatively to various viewpoints, it has also been revealed that said plasminogen activator has strong thrombus-resolving effect, leading to completion of this invention.

The following five prior art documents disclose plasminogen activators dissimilar from urokinase :
- on the one hand Chemical Abstracts, Vol. 76, n° 5, 1972, Abstract 22330d, discloses a plasminogen activator derived from the vascular tree of human corpses, whereas JBC, Vol. 254, n° 6, 1979, pages 1998-2003 deals with a plasminogen activator derived from the vessels of the lower extremities of corpses;
- Chemical Abstracts, Vol. 92, n° 17, 1980, Abstract 144679a and EP-A-0041766, on the other hand, are dealing with plasminogen activators originating from Melanoma cells; and

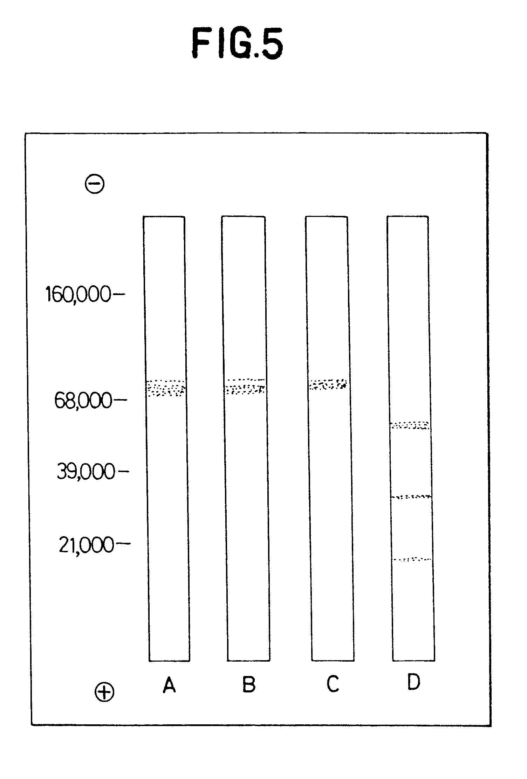

- finally Biochimica et Biophysica Acta, 717 (1982), pages 327-336 (issue dispatched on August 9, 1982) discloses a plasminogen activator thiocyanate-extracted from human kidneys having a molecular weight of 54 000 (page 332, col. 2, 8th line from bottom and figure 5 of said column).

The invention relates to a plasminogen activator having a molecular weight (m.w.) of about 70 000 which is quite dissimilar to other activators, as urokinase activator with a m.w. of 54 000 or 55 000 as occurs in BBA, 717, 1982 pp 327-336 and EP-A-0 099 126, since there are many different protease-like components in a kidney and a greater quantity of said components in a kidney when compared with a blood vessel.

A man skilled in the art would not be lead by the prior art to use a metal chelate Sepharose® column or another column recited at the end of claim 2 of the captioned application.

The advantage of such a column, especially of a metal chelate Sepharose® column is that an excellent repeated result is obtained for the products involved in the process of claim 2 and finally a plasminogen activator is obtained as claimed in claim 1 which is different from and better than the activators of the two just above-mentioned prior art documents.

Disclosure of the Invention :

An object of this invention is to provide a novel plasminogen activator derived from human kidney.

Another object of this invention is to provide a novel process for preparing the novel plasminogen activator.

A further object of this invention is to provide a novel thrombolytic agent having as ingredient said plasminogen activator and which has strong trombolytic effect.

The plasminogen activator derived from human kidney having the immunological property of not being adsorbed by anti-urokinase IgG-Sepharose® affinity chromatography, and of which the main protein band obtained by the isoelectric point electrophoresis has a pI in the range of 7 to 9, characterized by the fact that:

(1) the main protein band obtained by the sodium dodecyl sulfate-polyacrylamide gel electrophoresis has an apparent molecular weight of about 70,000 ± 5 000 and

(2) the plasminogen activator hydrolyses H-D-valyl-L-leucyl-L-lysine-p-niroanilide dihydrochloride and H-D-isoleucyl-L-prolyl-L-arginine-p-nitroanilide dihydrochloride; but does not hydrolyse Boc-L-valyl-L-prolyl-L-arginine-4-methylcoumaryl-7-amide, carbobenzoxyl-L-phenylalanyl-7-amide, L-prolyl-L-phenylalanyl-L-arginine-4-methylcoumaryl-7-amide and glutaryl-glycyl-L-arginine-4-methylcoumaryl-7-amide.

The above plasminogen activator is prepared by removing blood clot, connective tissue, lipids and the like from a human kidney in accordance with a method known per se in the art and subjecting the resultant human kidney tissue to an extraction treatment with a buffer or causing an $NH_4SCN$ (ammonium thiocyanate) solution to perfuse through a blood vessel to subject the blood vessel to an extraction; and then passing the resultant extract through a combination of columns to purify the same; this process being characterized by the fact that one of those columns is a metal chelate column.

The novel plasminogen activator of the present invention is capable of supplementing the immunological drawbacks of urokinase which has already been developed as a drug having thrombus-dissolving activity, and can thus be used as an active ingredient of a thrombolytic agent having strong thrombolytic effect.

Brief Description of the Drawings:

In the drawings:

Figure 1 is a zinc chelate-Sepharose® chromatogram of a plasminogen activator obtained in Example 1-(3);

Figure 2 is a concanavalin A-Sepharose® chromatogram of a plasminogen activator obtained in Example 1-(4);

Figure 3 is a CM-Sepharose® chromatogram of a plasminogen activator obtained in Example 1-(5);

Figure 4 shows results of the Sephacryl® S-200 gel filtration of a plasminogen activator obtained in Example 1-(6);

Figure 5 illustrates the SDS-polyacrylamide gel electrophoresis of a plasminogen activator according to this invention, which plasminogen activator had been isolated from kidneys. Letters A, B, C and D correspond respectively to the activator extracted from the kidneys and then purified, an activator extracted from vascular walls and then purified, an activator secreted from cultivated normal human diploid, and a commercially-available urokinase sample;

Figure 6 depicts identification results of plasminogen activators according to this invention, which had in advance been subjected to SDS-polyacrylamide gel electrophoreses. Letters A and B correspond to a purified plasminogen activator obtained in Example 1-(6), while letters C and D correspond to a crude extract obtained in Example 1-(3). A polyacrylamide gel was used to obtain the plasminogen activators A and C whereas a fibrin-agarose gel was employed to provide the plasminogen activators B and D.

Figure 7 are affinity chromatograms of urokinase and a kidney-originated plasminogen activator of this invention, both obtained by using antiurokinase IgG-Sepharose columns;

Best Mode for Carrying Out the Invention:

The process of this invention and properties of the novel activator obtained by the process will hereinafter be described in detail. Unless otherwise specifically indicated, all the procedures were carried out at 4°C.

Example 1:

Purification of plasminogen activator obtained from kidney:

(1) Extraction of plasminogen activator:

After mechanically removing blood clot, connective tissue and lipids from a human kidney which was free of any infections symptom (which had been stored at -70°C), acetone of -15°C was added and the kidney was then ground down in a homogenizer. The suspension was stirred at -15°C for 30 minutes and then allowed to stand at -20°C. The surface flotage was removed by decantation and defattening was repeated with acetone of -15°C. The resultant suspension was then filtered. The filtered cake was washed with acetone of -20°C and then dried. Thereafter, 100 g of the thus-defattened powder was suspended in 1 liter of a 1-mole $NH_4SCN$ solution buffered with a 0.02-mole Tris-HCl solution of pH 7.0 - 7.4. The resultant suspension was stirred. The suspension was separated into an extract and a precipitate by centrifugation. The precipitate was extracted again with the same 1 liter of the 1-mole $NH_4SCN$ solution. The extracts were combined to obtain a crude extract.

(2) DEAE-Sepharose® chromatography:

The crude extract obtained in the above procedure (1) was diluted with the same amount of a 0.02-mole Tris-HCl solution of pH 7.0 - 7.4. The thus-prepared sample was passed through a DEAE-Sepharose® column which had been equilibrated with a 0.02% solution of a nonionic surfactant, i.e., Tween 80 and a 0.02-mole Tris-HCl solution which contained 0.25 mole of $NH_4SCN$. The effluents of the column were combined, to which NaCl was added to make the final concentration be 1.0 mole. Its pH was adjusted to 7.4 with 1N-HCl.

(3) Zinc chelate-Sepharose® chromatography:

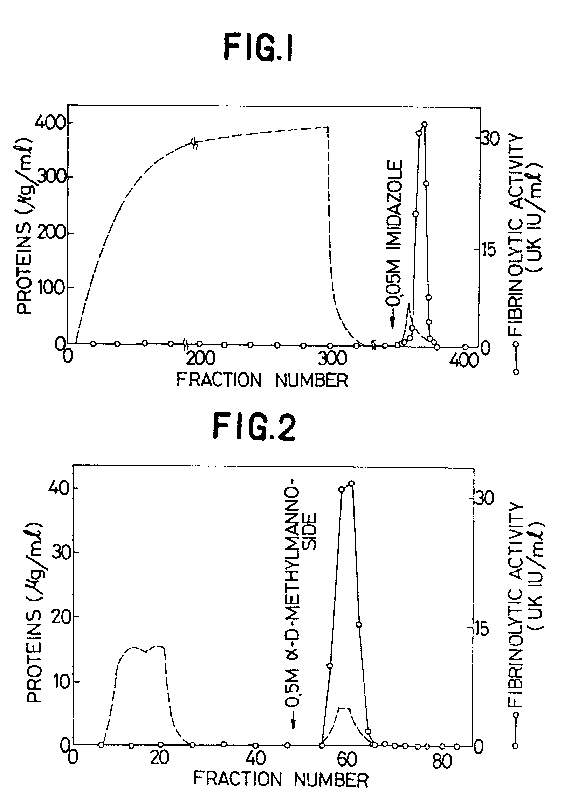

The effluents obtained in the above procedure (2) were passed through a zinc chelate-Sepharose® column which had been equilibrated with a 0.002-mole Tris-HCl solution which had a pH of 7.0 and contained 1.0 mole of NaCl, 0.25 mole of $NH_4SCN$ and 0.02 mole of Tween 80. After washing the column with the above equilibrating solution until the concentration of proteins in the eluate was lowered to 0.01 mg/ml. Thereafter, the column was washed further with a buffer the composition of which was identical to the above equilibrating solution except that the buffer contained no $NH_4SCN$ but 0.15 mole of NaCl. The column was then washed with a 0.05-mole imidazole solution, thereby eluting the tissue activator into the washings. The profile of the resulting chromatogram is shown in Figure 1.

(4) Concanavalin A-Sepharose® chromatography:

Fractions of the tissue activator, which fractions had been obtained in the above procedure (3), were passed through a concanavalin A-Sepharose® column. The column had in advance equilibrated with a 0.02-mole tris-HCl solution which had a pH of 7.4 and contained 0.15 mole of NaCl and 0.02% of Tween 80. After repeatedly washing the column with the equilibrating solution until the concentration of proteins in the eluate reached the base line, the tissue activator was eluted with an equilibrating solution which contained 0.5 mole of α-D-methylmannoside. Fractions containing the activator were combined and the pH of the resultant solution was adjusted to 4.5 with acetic acid. The profile of the resultant chromatogram is shown in Figure 2. Rijken et al reported that a linear gradient eluent of α-D-methylmannoside (0 - 0.6 mole) is effective when isolating a plasminogen activator from human uterus tissue [Biochim Biophys. Acta: 580, 140-153 (1979)]. When the linear gradient eluent of α-D-methylmannoside was employed, the elution profile of the activator activity was in conformity with that of proteins. Almost all the points of elution of the plasminogen activator were near the points of elution of the 0.35-mole α-D-methylmannoside solution and thus somewhat higher than those reported by Rijken et al.

(5) CM-Sepharose® chromatography:

Tissue activator-containing fractions obtained in the above procedure (4) were passed through a CM-Sepharose® column. The column had in advance been equilibrated with a 0.02-M acetic acid buffer which has a pH of 4.5 and contained 0.15 mole of NaCl and 0.02% of Tween 80.

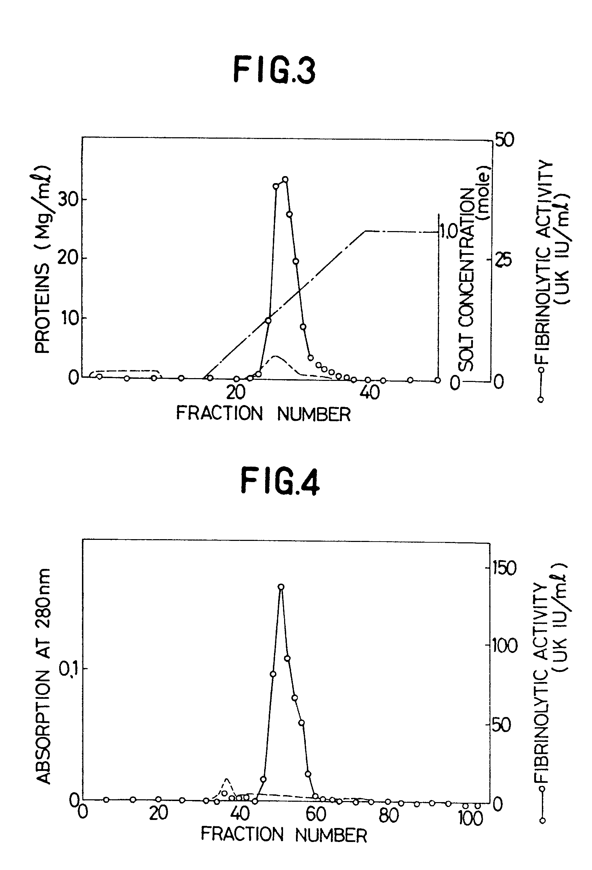

After washing the column with the equilibrating solution first of all, the column was developed with the same equilibrating solution except that the concentration of NaCl was linearly increased from 0.15 mole to 1.0 mole. The tissue activator was eluted near an NaCl concentration of 0.45 mole. The elution profile is illustrated in Figure 3. The tissue activator was adsorbed on the CM-Sepharose® in the equilibrating solution and about 50 - 60% of the loaded proteins was allowed to pass through the column. The tissue activator was eluted near an NaCl concentration of 0.45 mole. This is equivalent to about 40% elution of the proteins used. The specific activity was increased by about 2.4 times. Fractions containing the activator were combined together and subjected to an ultrafiltration through a Diaflo membrane PM 10 (product of Amicon Corporation), thereby to concentrate the same. The exchange of the activator sample with a buffer was conducted on a Sephadex G-25 (product of Pharmacia AB) column. The column had in advance been equilibrated with a 0.02-mole Tris-HCl solution which had a pH of 7.4 and contained 1.0 mole of $NH_4SCN$. The column was developed with a buffer which was identical to the equilibrating solution. Fractions, which contained the thus-eluted proteins, were combined together and then subjected to an ultrafiltration through a Diaflo membrane PM 10 to concentrate the same.

(6) Sephacryl® S-200 gel filtration:

A concentrated sample of the tissue activator was caused to pass through a Sephacryl S-200 (product of Pharmacia AB) column which had in advance been equilibrated with a 0.02-mole Tris-HCl solution which had a pH of 7.4 and contained 1.0 mole of $NH_4SCN$.

Effluents, which were fractions of the tissue activator, were stored at -70°C. The elution profile is shown in Figure 4.

Example 3:

Analysis of tissue plasminogen activator and results:
(1) Unless otherwise specifically indicated, the plasminogen activator was analyzed by the fibrin-agar plate method disclosed in Immunochemistry 9, 709-723 (1972). The fibrinolytic activity of the tissue activator was compared with that of urokinase and was expressed in terms of the international unit (UKIU). The standard curves showing the activity of serial dilutions of both plasminogen activator of this invention and urokinase were straight in the range of the lytic area of 70 - 240 $mm^2$. However, the gradient of the standard curve of the tissue activator was not completely consistent with that of urokinase. The activity of the tissue activator was determined by measuring the lytic areas of diluted samples within the range of 120 - 200 $mm^2$. The lytic area of 10 $\mu l$ or 1 IU/ml urokinase was 156 $mm^2$. The reproducibility of the fibrin-agar plate method was ± 6%. The enzymatic activity of the tissue activator was standardized by the enzymatic activity of urokinase and the percentage recovery of the tissue activator and its specific activity relative to UKIU/ml were measured after each of several purification stages.

The non-specific fibrin-resolving activity was measured on a fibrin-agar plate free of any plasminogen.

In some instances, the measurement was carried out in accordance with the method which used $^{125}$I-fibrin monomer trapped in a cellulose nitrate disc ("Selectgun" Type-BA; pore diameter: 0.2 $\mu m$) [Progress in Chemical Fibrinolysis and Thrombolysis, Vol. 3, 539-546 (1978), Raven Press, New York].

6

Fibrinogen which contained no human plasminogen was radiated by the method proposed by Hawker et al [J. Clin. Pathol. 29, 495-501 (1976)]. The specific activity of the labeled substrate was 0.018 mCi/mg fibrinogen. A sample mixture was incubated at 37°C and a 50 µl portion was sampled out at each of 1st and 7th hours. Released $^{125}$I was measured by an automatic gamma counter. The activity of each of the dilutions of the tissue activator and urokinase was expressed in terms of the percentage of released $^{125}$I relative to the total radioactivity by subtracting the released $^{125}$I.

(2) Affinity chromatography on anti-urokinase IgG-Sepharose column:

The urokinase antiserum was obtained from a goat which had been immunized by a subcutaneous administration of 1 mg of high-purity human urokinase (m.w. 33,000; specific activity: 202,400 IU/mg). The above-employed urokinase sample was the same as that analyzed above in accordance with the SDS-polyacrylamide gel electrophoresis.

The anti-urokinase serum showed only one precipitation curve when examined in accordance with the double immunodiffusion analysis. IgG fractions of antiurokinase goat serum and unimmunized goat serum were prepared by precipitating with a saturated 33% ammonium sulfate solution. They were purified further by DEAE-Sepharose chromatography.

The anti-urokinase IgG (50 µg/ml) added to a fibrin plate inhibited completely the activity of the commercial urokinase sample which was used as an antigen (5,000 IU/ml). These IgG samples (20 mg/ml) individually developed a single precipitation curve when the immunoelectrophoresis was applied using anti-goat serum and rabbit serum.

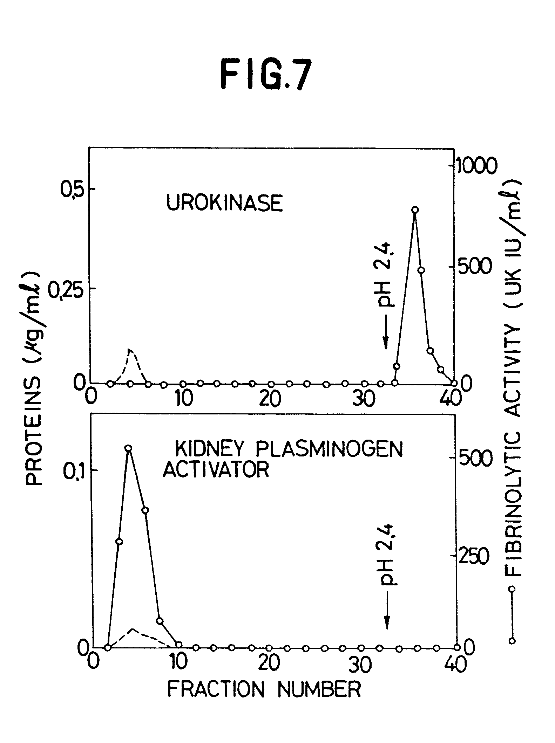

Fifty milligrams of the anti-urokinase or normal IgG sample was coupled with 15 ml of cyanogen bromide-activated Sepharose® CL-4B in accordance with the method proposed by Cuatrecasas [J. Biol. Chem. 245, 3059-3065, (1970)]. The gel was washed with a 0.02-mole Tris-HCl solution, which had a pH of 7.4 and contained 1.0 mole of NaCl, of a volume at least 20 times the volume of the gel. Chromatograms of the tissue activator and urokinase are shown in Figure 7.

(3) Adsorption of the tissue activator and urokinase on fibrin-Sepharose:

According to the aforementioned Cuatrecasas's method, 200 mg of fibrinogen which was free of human plasminogen was coupled at pH 8.3 with 20 g of cyanogen bromide-activated Sepharose® CL4B. The thus-coupled fibrinogen was then activated by an addition of thrombin (1 Unit/ml) in accordance with the method proposed by Reeme et al [Thromb. Res. 2, 137-143 (1973)]. Thereafter, it was washed with a 0.02-mole Tris-HCl solution which had a pH of 7.4 and contained 0.15 mole of NaCl, 0.1% of Triton X-100, and 1 micromole of Aprotinin (product of Beyer AG). The gel, which had been loaded in the column, was washed with 2 mole of $NH_4SCN$ dissolved in a buffer, followed by an addition of a urokinase or tissue activator sample (which was obtained from the stage (6) of Example 1) in a buffer to the column. After washing the column, the column was eluted with a 2-mole $NH_4SCH$ solution. The activity of the plasminogen activator was measured in accordance with a method which employed the concentration of proteins in the eluate and a chromogenic substrate S-2288.

(4) Other methods:

The quantitative analyses of proteins were conducted using the method proposed by Lowry et al, which method used bovine serum albumine as a standard protein [J. Biol. Chem. 193, 265-275 (1951)]. Solubilized proteins in Tween® 80 were quantatively analyzed by the method proposed by Shiu et al [J. Biol. Chem. 249, 7902-7911 (1974)], after the sediment was removed by centrifugal separation. In some instances, a more accurate method making use of fluorescamine was used [Arch. Biochem. Biophys. 155, 213-220 (1973)]. Eluates obtained after the Sephacryl® S-200 chromatography were monitored using an LKB Uvicord II detector and recorder, which detector was equipped with a Hitachi Spectrophotometer Model 200-200.

The SDS-polyacrylamide gel electrophoresis was conducted in accordance with the method proposed by Weber et al [J. Biol. Chem. 244, 4406-4412 (1972)]. Each protein sample was incubated at room temperature for 30 minutes in a 1.0% SDS solution and then subjected to electrophoresis in a 5% polyacrylamide gel. In some instances, proteins were reduced with a 1% solution of 2-mercaptoethanol. The gel dyed a protein with Coomassie Brilliant Blue or a glycoprotein with periodic acid-Schiff's reagent. In order to measure the activity of the plasminogen activator, the SDS-polyacrylamide gel electrophoresis was conducted in a 5% polyacrylamide gel in accordance with the slab gel system. After conducting the electrophoresis, the slab gel was washed at room temperature for 2 hours with a 0.01-mole phosphoric acid buffer which had a pH of 7.4 and contained 0.5 mole of NaCl and 2% of Tween® 80. The gel was then stacked with a 1% agarose gel which contained 0.2% of human fibrinogen (either rich in or free of plasminogen) and 0.5 unit of thrombine. The contents were then incubated for 1 - 7 hours at 37°C in a constant-humidity chamber and the agarose and polyacrylamide gel were dyed with Coomassie Brilliant Blue. The apparent molecular weight of the protein was measured by comparing its mobility with the mobility of the standard protein in accordance with the aforementioned method proposed by Weber et al.

The measurement of the isoelectric point of the thus-purified tissue activator was carried out in a 7.5% polyacrylamide gel containing 8 moles of urea and 1% of an ampholyte in accordance with the method proposed by Mertz et al [Biochem. Biophys. Res. Commun. 49, 84-91 (1972)]. The sample was solubilized in an 8-mole urea solution which contained 2 millimoles of $\overline{EDTA}$ and 1% of an ampholyte. Its pH was determined by the method proposed by Finlayson et al [Anal. Biochem. 40, 292-311 (1971)].

The thus-purified tissue activator was tested with various fluorescent substrates, after adding 50 µl of the tissue activator sample in a 0.02-mole Tris-HCl solution having a pH of 8.0 and containing 0.1 mole of NaCl and 0.02% of Tween 80 and 2.0 ml of the substrate in the same buffer. The following materials were used as fluorescent substrates:

Boc-L-Valyl-L-prolyl-L-arginine-4-methylcumaryl-7-amide;
Carbobenzoxy-L-phenylalanyl-L-arginine-4-methylcumaryl-7-amide;
L-Prolyl-L-phenylalanyl-L-arginine-4-methylcumaryl-7-amide; and
Glutaryl-glycyl-L-arginine-4-methylcumaryl-7-amide.

The initial release of 7-amino-4-methylcumarine (AMC) was measured by a fluorometer, at 37°C in a Shimadzu Fluorospectrophotometer Model RF-520. The fluorometer had been set in such a manner that a radiation of light of 380 nm would have permitted to emit an excited fluorescent light of 460 nm. The fluorometer was also adjusted so that a 0.2-mole AMC solution in a 0.1% DMSO solution gave a relative fluorescent unit of 1.0. The hydrolytic activity of the purified tissue activator was measured with a chromogenic substrate, after adding 200 µl of a tissue activator sample, 200 µl of a 0.1-mole Tris-HCl solution having a pH of 8.0 and containing 0.1 mole of NaCl and 0.02% of Tween 80, and 200 µl of a 3-millimole S-2251 solution or a 1-millimole S-2288 solution. The initial release of p-nitroaniline was measured by a spectrophotometer, at 405 nm and 37°C in a glass cell. The initial velocity of the hydrolytic activity was expressed in terms of ΔmA/min. for the chromogenic substrate and Δ%/min. for the fluorescent substrate. The inhibitory effects of the tissue activator when treated respectively with diisopropyl fluorophosphate, iodoacetamide, soybean trypsin inhibitor and L-arginine aprotinin were tested with the chromogenic substrate S-2288. The purified tissue activator samples were incubated at 37°C for 1 hour after adding the inhibitors therein. A 200 µl portion was taken out from each mixture, followed by an addition of 200 µl of a buffer and 1 millimole of the substrate. By comparing ΔA/min. values with one another, the inhibitory effect of each reagent was measured.

(5) Samples were purified by the method of Example 1 and then analyzed in accordance with the analytic method described in Example 2. Results are shown in Table 2.

Table 2  Purification of Tissue Plasminogen Activator Obtained from Kidneys

| Purification stage | Total activity (UKIU) | Specific activity (UKIU/mg) | Purification degree | Percentage recovery (%) |
|---|---|---|---|---|
| 1. NH$_4$SCN extraction | 8,600 | 0.8 | 1 | 100 |
| 2. DEAE-Sepharose® | 7,800 | 2.0 | 2.4 | 90 |
| 3. Zinc Chelate-Sepharose® | 6,200 | 300 | 357 | 72 |
| 4. Concanavlin A-Sepharose® | 4,300 | 5,280 | 6,286 | 50 |
| 5. CM-Sepharose® | 3,500 | 12,300 | 14,600 | 41 |
| 6. Sephacryl S-200® | 2,700 | 25,700 | 30,600 | 31 |

Example 4:

Properties of the tissue plasminogen activator of this invention:

(1) Molecular weight:

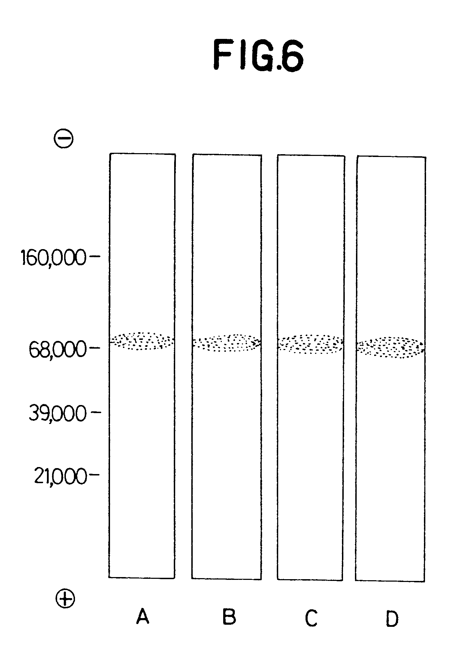

The molecular weight of the purified tissue activator obtained in Example 1 was measured by the SDS-polyacrylamide gel electrophoresis. As a result, the molecular weight of the kidney-originated activator was about 72,000. These tissue activators were positive to the periodic acid-Schiff's reagent, thereby indicating the presence of sugar chains. In the case of the kidney-originated activator for instance, the mobility of the main band of the purified tissue activator as measured by the electrophoresis was identical to the mobility of the main band of resolved fibrin of a crude extract, when the crude extract and purified tissue activator were respectively analyzed by the SDS-polyacrylamide gel electrophoresis and the resulting gels were respectively developed on fibrin-agar plates. This indicates that the apparent molecular weight was 70,000 (see, Figure 6). The band of resolved fibrin was attributed to the presence of plasminogen in the fibrin-agar plate.

From the above finding, it is understood that the crude extract obtained from human kidney tissue contains a plasminogen activator and the plasminogen activator consists principally of that having an apparent molecular weight of about 70,000.

(2) Isoelectric point:

As the isoelectric points of purified activator, the main peak appeared at 8.2 in the case of a kidney-originated activator. After measurement of each isoelectric point, its activity was measured on a fibrin-agar plate which was in the form of a thin-film gel (thickness: 1.5 mm). The activity of the activator was found respectively near its isoelectric point, i.e., pH 8.2.

(3) Immunological differences between tissue activators and urokinase:

In order to study whether the activators obtained respectively from kidney tissue and the wall of a blood vessel have the same antigenicity as urokinase, the elution profile of proteins obtained by the antiurokinase IgG-Sepharose affinity chromatographic treatment of the purified activator of Example 1 was compared with the elution profile of urokinase. Results are shown in Figure 7. As seen from Figure 7, urokinase was adsorbed strongly on the column and almost all the loaded activity was eluted with a 0.1-mole glycine-HCl solution which contained 0.15 mole of NaCl and 0.1% of Triton® X-100. The protein contained in the effluents was serum albumin which had been incorporated in the commercial urokinase used in this experiment. On the other hand, the purified activator was passed through the column. It was however not adsorbed and neither protein nor activity was eluted with a glycine solution of pH 2.4. When a partially-purified sample was poured in the same column, about 15% of the poured activity was adsorbed on the column and eluted with a glycine solution of pH 2.4. On the basis of these findings, it is readily envisaged that the plasminogen activator of this invention is a tissue activator which is immunologically dissimilar to urokinase and the crude extract obtained from kidneys contains, as a minor ingredient, urokinase or an urokinase-like activator.

(4) Affinity of tissue activators to fibrin-Sepharose®:

The affinity of each of urokinase and a tissue activator to insolulized fibrin monomer was investigated using fibrin-Sepharose® columns.

The tissue activator according to this invention was adsorbed in its entirety on the fibrin-Sepharose® column. It was then eluted with a 2-mole $NH_4SCN$ solution and at least 90% of the total poured activity was recovered. On the other hand, almost the whole active ingredients of urokinase were allowed to pass through the column. The activity of the activator was thus not found in the eluate obtained with a 2-mole $NH_4SCN$ solution from the column. As has been mentioned above, the tissue activator of this invention which was obtained from human kidneys has strong affinity to fibrin-Sepharose.

(5) Specificity of synthetic substrates to tissue activators:

The specificity of certain synthetic substrates to the activator obtained in Example 1-(6) was studied. The following substrates were employed.

For plasmin, H-D-valyl-L-leucyl-L-lysine-p-nitroanilide dihydrochloride (S-2251);

For natural thrombine, Boc-L-valyl-L-prolyl-L-arginine-4-methylcoumaryl-7-amide (3093-V);

For plasma, carbobenzoxy-L-phenylalanyl-L-arginine-4-methylcoumaryl-7-amide (3095-V);

For urine kallikrein, L-prolyl-L-phenylalanyl-L-arginine-4-methylcoumaryl-7-amide (3096-V);

For urokinase, glutamyl-glycyl-L-arginine-4-methylcoumaryl-7-amide (3097-V); and

For tissue plasminogen activator, H-D-isoleucyl-L-prolyl-L-arginine-p-nitroanilide dihydrochloride (S-2288).

The tissue activator of this invention did not show hydrolytic activity to any substrates other than S-2251 and S-2288. The activity (100 UKIU/ml) of the tissue activator according to this invention was 88 ΔmA/min. against S-2251 and 70 ΔmA/min. against S-2288.

The activator of this invention showed some characteristic properties when treated with certain inhibitors. The amide-decomposing activity of the activator of this invention against S-2288 was not inhibited where 50 KIU/ml of Aprotinin, 10 millimoles or iodoacetamide or 50 $\mu$g/ml of soybeen trypsin was used as an inhibitor. However, it was inhibited up to 26% by 100 millimoles of L-arginine and up to 98% by 5 millimoles of diisopropyl fluorophosphate.

(6) Stability:

In the case of the plasminogen activator according to this invention, no activity reduction was observed at all even two weeks later when stored at 4°C in a buffer of pH 7.4. The same result was also obtained even when 1 mole of $NH_4SCN$, 0.02% of Tween® 80 or 0.1 mole of NaCl was incorporated in the buffer.

The stability of the plasminogen activator of this invention was investigated at 4°C for 24 hours in buffers which had pH levels in the range of 1 - 10 and contained 0.5 mole of NaCl and 0.02% of Tween 80. As a result, it was found that the plasminogen activator of this invention was stable within the pH range of 2 - 10 but an activity reduction of 70% occurred at pH 1.0.

As has been described in detail, the activator of this invention is a novel plasminogen activator which is different from urokinase and plays an important role in the control of recovery of a tissue, including the decomposition of fibrin deposits on inner and outer vascular walls at a damaged part.

In accordance with the above-described preparation process of the activator to which process the present invention also relates, the tissue activator can be isolated in a highly purified form. The tissue activator shows a specific activity as much as 30,000 - 45,000 times the specific activity of a human kidney dried with acetone.

The thrombolytic agent according to this invention will naturally become apparent from the following Examples. It should however be borne in mind that the plasminogen activator, which was used as an ingredient, had been derived from normal cells. A plasminogen activator originated from malignant tumor cells is known to have thrombolytic activity. However, the safety of a thrombolytic agent according to this invention is believed to have already been assured owing to its origin, when, in view of its manner of application as a drug to be administered to men, one takes into consideration various potential risks in its administration over a long period of time or at a high dosage level.

Since the plasminogen activator as an ingredient of a thrombolytic agent according to this invention has specific affinity, which is different from that of urokinase but similar to that of the normal plasminogen activator obtained from blood, to fibrin, the thrombolytic agent can bring about fully satisfactory results when employed for the treatment of thrombosis. In addition, the thrombolytic agent according to this invention is free from such side effects as internal bleeding and anaphylaxis shock. Accordingly, the thrombus resolvent according to this invention is an extremely effective therapeutic drug.

The thrombolytic agent of this invention may be administered at suitable dosage levels to patients and has potency accompanied with slight or no side effects and effective thrombolytic activity. Namely, the thrombolytic agent according to this invention may be administered in much the same way as commonly-employed other drugs of the same type. For example, the plasminogen activator which is an active ingredient may be dissolved in a physiological saline containing 5% of human serum albumin and may then be administered by injection. Here, it is preferred to limit each dosage to 10,000 IU (about 100 $\mu$g) as measured in terms of urokinase.

The thrombolytic agent according to this invention may be used to treat acute and chronic thrombus occulusions of various blood vascular beds as encountered in cases of deep venous thrombosis, pulmonary embolism, myocardial infraction, arterial occlusion, extracorporeal circulation and arteriovenous occlusion.

**Claims**

**1.** A plasminogen activator derived from human kidney, having the immunological property of not being adsorbed by anti-urokinase IgG-Sepharose® affinity chromatography, and of which the main protein band obtained by the isoelectric point electrophoresis has a pI in the range of 7 to 9, characterized by the fact that :

(1) the main protein band obtained by the sodium dodecyl sulfate-polyacrylamide gel electrophoresis has an apparent molecular weight of about 70.000 ± 5000 and

(2) the plasminogen activator hydrolyses H-D-valyl-L-leucyl-L-lysine-p-nitroanilide dihydrochloride and H-D-isoleucyl-L-prolyl-L-arginine-p-nitroanilide dihydrochloride; but does not hydrolyse Boc-L-valyl-L-prolyl-L-arginine-4-methylcoumaryl-7-amide, carbobenzoxyl-L-phenylalanyl-7-amide, L-propyl-L-phenylalanyl-L-arginine-4-methylcoumaryl-7-amide                and                glutaryl-glycyl-L-arginine-4-methylcoumaryl-7-amide.

2. A process for preparing a plasminogen activator derived from human kidney, having the immunological property of not being adsorbed by anti-urokinase IgG-Sepharose® affinity chromatography, and of which the main protein band obtained by the isoelectric point electrophoresis has a pI in the range of 7 to 9, characterized by the fact that :

(1) the main protein band obtained by the sodium dodecyl sulfate-polyacrylamide gel electrophoresis has an apparent molecular weight of about 70.000 ± 5000 and

(2) the plasminogen activator hydrolyses H-D-valyl-L-leucyl-L-lysine-p-nitroanilide dihydrochloride and H-D-isoleucyl-L-prolyl-L-arginine-p-nitroanilide dihydrochloride; but does not hydrolyse Boc-L-valyl-L-prolyl-L-arginine-4-methylcoumaryl-7-amide, carbobenzoxyl-L-phenylalanyl-7-amide, L-propyl-L-phenylalanyl-L-arginine-4-methylcoumaryl-7-amide and glutaryl-glycyl-L-arginine-4-methylcoumaryl-7-amide;

which process comprises :

subjecting a human kidney to an extraction with an ammonium thiocyanate solution; and, then,

passing the resultant extract through a combination of columns to purify the same, characterized by the fact that one of these columns is a metal chelate column.

3. A process according to claim 2, wherein, the human kidney is subjected to the extraction with an ammonium thiocyanate solution buffered with Tris-HCl at a pH in the range of 7.0 to 7.4.

4. A thrombolytic agent containing, as an active ingredient, a plasminogen activator derived from human kidney, having the immunological property of not being adsorbed by anti-urokinase IgG-Sepharose® affinity chromatography, and of which the main protein band obtained by the isoelectric point electrophoresis has a pI in the range of 7 to 9, characterized by the fact that :

(1) the main protein band obtained by the sodium dodecyl sulfate-polyacrylamide gel electrophoresis has an apparent molecular weight of about 70.000 ± 5000 and

(2) the plasminogen activator hydrolyses H-D-valyl-L-leucyl-L-lysine-p-nitroanilide dihydrochloride and H-D-isoleucyl-L-prolyl-L-arginine-p-nitroanilide dihydrochloride; but does not hydrolyse Boc-L-valyl-L-prolyl-L-arginine-4-methylcoumaryl-7-amide, carbobenzoxyl-L-phenylalanyl-7-amide, L-propyl-L-phenylalanyl-L-arginine-4-methylcoumaryl-7-amide and glutaryl-glycyl-L-arginine-4-methylcoumaryl-7-amide.

5. A thrombolytic agent according to claim 4, wherein the plasminogen activator has been derived from normal diploid cells.

6. A thrombolytic agent according to claim 4 or 5, which is useful in the treatment of acute or chronic occlusion of an artery or vein by thrombus.

**Patentansprüche**

1. Plasminogen-Aktivator aus menschlicher Niere mit der nicht durch eine Anti-Urokinase IgG-Sepharose® -Affinitätschromatographie adsorbiert werdenden immunologischen Eigenschaft, wobei das durch isoelektrische Punktelektrophorese erhaltene Hauptproteinband einen pI-Wert im Bereich von 7 bis 9 aufweist,

dadurch gekennzeichnet,

(1) daß das durch Sodiumdodecylsulfat-Polyacrylamidgel-Elektrophorese erhaltene Hauptproteinband ein scheinbares Molekulargewicht von etwa 70.000 +/- 5000 aufweist und

(2) daß der Plasminogenaktivator H-D-Valyl-L-Leucyl-L-Lysin-p-Nitroanilid-Dihydrochlorid und H-D-Isoleucyl-L-Prolyl-L-Arginin-p-Nitroanilid-Dihydrochlorid hydrolisiert; aber Boc-L-Valyl-L-Prolyl-L-Arginin-4-Methylcoumaryl-7-Amid, Carbobenzoxyl-L-Phenylalanyl-7-Amid, L-Propyl-L-Phenylalanyl-L-Arginin-4-Methylcoumaryl-7-Amid und Glutaryl -Glycyl-L-Arginin-4-Methylcoumaryl-7-Amid nicht hydrolisiert.

2. Verfahren zur Herstellung eines Plasminogenaktivators aus menschlicher Niere mit der nicht durch eine Anti-Urokinase IgG-Sepharose® -Affinitätschromatographie adsorbiert werdenden immunologischen Eigenschaft, wobei das durch isoelektrische Punktelektrophorese erhaltene Hauptproteinband einen pI-Wert im Bereich von 7 bis 9 aufweist, dadurch gekennzeichnet,

(1) daß das durch Sodiumdodecylsulfat-Polyacrylamidgel-Elektrophorese erhaltene Hauptproteinband ein scheinbares Molekulargewicht von etwa 70.000 +/- 5000 aufweist und

(2) daß der Plasminogenaktivator H-D-Valyl-L-Leucyl-L-Lysin-p-Nitroanilid-Dihydrochlorid und H-D-Isoleucyl-L-Prolyl-L-Arginin-p-Nitroanilid-Dihydrochlorid hydrolisiert; aber Boc-L-Valyl-L-Prolyl-L-Arginin-4-Methylcoumaryl-7-Amid, Carbobenzoxyl-L-Phenylalanyl-7-Amid, L-Propyl-L-Phenylalanyl-L-Arginin-4-Methylcoumaryl-7-Amid und Glutaryl -Glycyl-L-Arginin-4-Methylcoumaryl-7-Amid nicht hydrolisiert,

wobei das Verfahren folgende Schritte aufweist:

Unterziehen einer menschlichen Niere einer Extraktion mit einer Ammonium-Thiocyanat-Lösung; und dann

Passierenlassen des resultierenden Extraktes durch eine Kombination von Kolonnen, um dasselbe zu reinigen, dadurch gekennzeichnet, daß eine dieser Kolonnen eine Metallchelatkolonne ist.

**3.** Verfahren nach Anspruch 2, wobei die menschliche Niere einer Extraktion mit einer Ammonium-Thiocyanat-Lösung gepuffert mit Tris-HCl bei einem pH-Wert im Bereich von 7 bis 7,4 unterzogen wird.

**4.** Thrombolytisches Mittel enthaltend als aktives Ingredienz einen Plasminogenaktivator aus menschlicher Niere mit der nicht durch eine Anti-Urokinase IgG-Sepharose® -Affinitätschromatographie adsorbiert werdenden immunologischen Eigenschaft, wobei das durch isoelektrische Punktelektrophorese erhaltene Hauptproteinband einen pI-Wert im Bereich von 7 bis 9 aufweist, dadurch gekennzeichnet,

(1) daß das durch Sodiumdodecylsulfat-Polyacrylamidgel-Elektrophorese erhaltene Hauptproteinband ein scheinbares Molekulargewicht von etwa 70.000 +/- 5000 aufweist und

(2) daß der Plasminogenaktivator H-D-Valyl-L-Leucyl-L-Lysin-p-Nitroanilid-Dihydrochlorid und H-D-Isoleucyl-L-Prolyl-L-Arginin-p-Nitroanilid-Dihydrochlorid hydrolisiert; aber Boc-L-Valyl-L-Prolyl-L-Arginin-4-Methylcoumaryl-7-Amid, Carbobenzoxyl-L-Phenylalanyl-7-Amid, L-Propyl-L-Phenylalanyl-L-Arginin-4-Methylcoumaryl-7-Amid und Glutaryl -Glycyl-L-Arginin-4-Methylcoumaryl-7-Amid nicht hydrolisiert.

**5.** Thrombolytisches Mittel nach Anspruch 4, wobei der Plasminogenaktivator aus normalen Diploidzellen gewonnen worden ist.

**6.** Thrombolytisches Mittel nach Anspruch 4 oder 5, welches geeignet zur Behandlung einer akuten oder chronischen Okklusion einer Arterie oder Vene durch einen Thrombus ist.

**Revendications**

**1.** Activateur de plasminogène dérivé de rein humain, ayant la propriété immunologique de ne pas être adsorbé par chromatographie d'affinité anti-urokinase IgG-Sepharose®, et dont la bande protéique principale obtenue par l'électrophorèse du point isoélectrique a un pI dans la gamme de 7 à 9, caractérisé par le fait que:

(1) la bande protéique principale obtenue par l'électrophorèse sur gel de sodium dodécyl-sulfate-polyacrylamide a un poids moléculaire apparent d'environ 70.000 ± 5000 et

(2) l'activateur de plasminogène hydrolyse le dihydrochlorure de H-D-valyl-L-leucyl-L-lysine-p-nitroanilide et le dihydrochlorure de H-D-isoleucyl-L-prolyl-L-arginine-p-nitroanilide; mais n'hydrolyse pas le Boc-L-valyl-L-propyl-L-arginine-4-méthylcoumaryl-7-amide, le carbobenzoxyl-L-phénylalanyl-7-amide, le L-propyl-L-phénylalanyl-L-arginine-4-méthylcoumaryl-7-amide et le glutaryl-glycyl-L-arginine-4-méthylcoumaryl-7-amide.

**2.** Procédé pour la préparation d'un activateur de plasminogène dérivé de rein humain, ayant la propriété immunologique de ne pas être adsorbé par chromatographie d'affinité anti-urokinase IgG-Sepharose®, et dont la bande protéique principale obtenue par l'électrophorèse du point isoélectrique a un pI dans la gamme de 7 à 9, caractérisé par le fait que:

(1) la bande protéique principale obtenue par l'électrophorèse sur gel de sodium dodécyl-sulfate-polyacrylamide a un poids moléculaire apparent d'environ 70.000 ± 5000 et

(2) l'activateur de plasminogène hydrolyse le dihydrochlorure de H-D-valyl-L-leucyl-L-lysine-p-nitroanilide et le dihydrochlorure de H-D-isoleucyl-L-prolyl-L-arginine-p-nitroanilide; mais n'hydrolyse pas le Boc-L-valyl-L-propyl-L-arginine-4-méthylcoumaryl-7-amide, le carbobenzoxyl-L-phénylalanyl-7-amide, le L-propyl-L-phénylalanyl-L-arginine-4-méthylcoumaryl-7-amide et le glutaryl-glycyl-L-arginine-4-méthylcoumaryl-7-amide;

ce procédé comprenant:

la soumission d'un rein humain à une extraction avec une solution d'ammonium thiocyanate; et, ensuite,

le passage de l'extrait résultant à travers une combinaison de colonnes pour le purifier, caractérisé par le fait que l'une de ces colonnes est une colonne de métal chélaté.

3. Procédé selon la revendication 2, dans lequel le rein humain est soumis à une extraction avec une solution d'ammonium thiocyanate tamponnée avec du Tris-HCl à un pH dans la gamme de 7,0 à 7,4.

4. Agent thrombolytique contenant, en tant qu'ingrédient actif, un activateur de plasminogène dérivé de rein humain, ayant la propriété immunologique de ne pas être adsorbé par chromatographie d'affinité anti-urokinase IgG-Sepharose®, et dont la bande protéique principale obtenue par l'électrophorèse du point isoélectrique a un pI dans la gamme de 7 à 9, caractérisé par le fait que:

(1) la bande protéique principale obtenue par l'électrophorèse sur gel de sodium dodécyl-sulfate-polyacrylamide a un poids moléculaire apparent d'environ 70.000 ± 5000 et

(2) l'activateur de plasminogène hydrolyse le dihydrochlorure de H-D-valyl-L-leucyl-L-lysine-p-nitroanilide et le dihydrochlorure de H-D-isoleucyl-L-prolyl-L-arginine-p-nitroanilide; mais n'hydrolyse pas le Boc-L-valyl-L-propyl-L-arginine-4-méthylcoumaryl-7-amide, le carbobenzoxyl-L-phénylalanyl-7-amide, le L-propyl-L-phénylalanyl-L-arginine-4-méthylcoumaryl-7-amide et le glutaryl-glycyl-L-arginine-4-méthylcoumaryl-7-amide.

5. Agent thrombolytique selon la revendication 4, dans lequel l'activateur de plasminogène a été dérivé de cellules diploïdes normales.

6. Agent thrombolytique selon la revendication 4 ou 5, qui est utile dans le traitement d'occlusion aiguë ou chronique par thrombose d'une artère ou d'une veine.

# FIG.1

# FIG.2

# FIG.3

# FIG.4

# FIG.5

# FIG.6

FIG.7